# EUROPEAN PATENT SPECIFICATION

(11) **EP 1 346 740 B1**
(45) Date of publication and mention of the grant of the patent: **08.09.2010**
(21) Application number: 03075799.1
(22) Date of filing: 19.03.2003
(51) Int. Cl.: A61M 5/32, A61M 5/50, A61M 25/06

(54) **Needle device**
Nadelvorrichtung
Dispositif d'aiguille

(30) Priority: 19.03.2002 US 365436 P
(43) Date of publication of application: 24.09.2003
(73) Proprietor: Becton Dickinson and Company, Franklin Lakes, New Jersey 07417 (US)
(72) Inventor: Bressler, Peter W., Philadelphia, PA 19147 (US); Coleman, John, Philadelphia, PA 19127 (US); Turpault, Mathieu, Berwyn, PA 19312 (US)
(74) Representative: 't Jong, Bastiaan Jacobus

(56) References cited:
- EP-A- 1 163 919
- DE-U- 20 103 363
- US-A- 5 743 888
- US-A- 5 755 699
- US-A- 5 951 525

## Description

### BACKGROUND OF THE INVENTION

### 1. Field of the Invention

The present invention relates to a needle assembly for a blood collection set according to the preamble of claim 1. Such an assembly is known from e.g. DE-U-201 03 363, US-A-5 755 699, EP-A-1 163 919 and US-A-5 743 888.

### 2. Description of Related Art

Disposable medial devices having medical needles are used for administering medication or withdrawing fluid from the body of a patient. Such disposable medical devices typically include blood collecting needles, fluid handling needles, and assemblies thereof. Current medical practice requires that fluid containers and needle assemblies used in such devices be inexpensive and readily disposable. Consequently, existing blood collection devices typically employ some form of durable, reusable holder on which detachable and disposable medical needles and fluid collection tubes may be mounted. A blood collection device of this nature may be assembled prior to use and then disassembled after use. Thus, these blood collection devices allow repeated use of a relatively expensive holder upon replacement of relatively inexpensive medical needles and/or fluid collection tubes. In addition to reducing the cost of collecting blood specimens, these blood collection devices help minimize the production of hazardous waste material.

A blood collection device or intravenous (IV) infusion device typically includes a needle cannula having a proximal end, a pointed distal end, and a lumen extending therebetween. The proximal end of the needle cannula is securely mounted in a plastic hub defining a central passage that communicates with the lumen extending through the needle cannula. A thin, flexible thermoplastic tube is connected to the hub and communicates with the lumen of the needle cannula. The end of the plastic tube remote from the needle cannula may include a fixture for connecting the needle cannula to a blood collection tube or other receptacle. The specific construction of the fixture will depend upon the characteristics of the receptacle to which the fixture is to be connected.

In order to reduce the risk of incurring an accidental needle-stick wound, protection of used needle cannulas becomes important. With concern about infection and transmission of diseases, methods and devices to enclose or cover the used needle cannula have become very important and in great demand in the medical field. For example, needle assemblies commonly employ a safety shield that can be moved into shielding engagement with a used needle cannula to minimize risk of an accidental needle stick.

Some needle safety shields are referred to as "tip guards" and include a small rigid guard that may be telescoped along the length of the needle cannula and extended over the pointed distal end of the needle cannula for protection. Such conventional tip guards may include some form of tether for limiting the travel of the tip guard to the length of the needle cannula. An example of the foregoing is disclosed by U.S. Patent No. 5,176,655 to McCormick et al. The McCormick et al. patent discloses the use of flexible loop-like straps for limiting the distal movement of a tip guard.

Needle shields that incorporate movable tip guards are typically manually actuated. For example, U.S. Patent Nos. Re 36,447 and Re 36,398, both to Byrne et al., disclose a safety device for a hypodermic needle that includes a plastic sheath, which is used to cover the puncture tip of the needle. The plastic sheath incorporates a thumb guard, which the user of the safety device may grasp to move the plastic sheath to a position covering the puncture tip of the needle cannula. U.S. Patent No. 5,951,525 to Thorne et al. discloses a manually operated safety needle apparatus that includes two pairs of opposed legs adapted to move the tip guard of the apparatus to a position covering the used needle cannula. U.S. Patent Nos. 5,562,637 and 5,562,636, both to Utterburg, disclose a rectangular needle protector sheath for use with a needle cannula that may be extended over the needle cannula after its use. Other prior art devices, such as those disclosed by U.S. Patent Nos. 5,290,264 to Utterberg and 5,192,275 to Burns provide "grippable" members attached to the tip guards to facilitate moving the tip guards to a position covering the puncture tip of a needle cannula. In addition to providing gripping members for moving the tip guards, prior art devices in this area often include flexible wings, which are used as means for securing the needle assemblies to the body of a patient during a medical procedure. Examples of "winged" needle assemblies may found in U.S. Patent Nos. 5,120,320 to Fayngold; and 5,154,699; 5,088,982; and 5,085,639 all to Ryan. Other prior art in this area includes U.S. Patent Nos. 5,266,072 and 5,112,311, both to Utterberg et al., which also disclose guarded winged needle assemblies.

Conventional tip guards, such as those discussed hereinabove, often include a structure that lockingly engages over the pointed distal end of the used needle cannula to prevent a re-exposure of the needle cannula. The structure for preventing the re-exposure of the needle cannula may include a metallic spring clip or a transverse wall formed integrally with one end of the tip guard. An example of a metallic spring clip is disclosed by the McCormick et al. patent discussed previously.

Conventional tip guards, such as those discussed hereinabove, often further require extensive mechanics for positioning the tip guard over the needle cannula. This results in complex arrangements that are costly to manufacture and assemble. Additionally, operation of the needle assemblies to move the tip guard into the proper position over the pointed distal end of the needle cannula requires substantial manual manipulation by the user of the device, exposing the user to potential needle-stick wounds.

In view of the foregoing, a need exists for a blood collection set including a shieldable needle assembly that achieves secure and effective shielding of a used needle cannula which is simple and inexpensive to manufacture and easy to operate.

### SUMMARY OF THE INVENTION

The present invention as claimed is directed to a needle assembly for use in a blood collection set as claimed in claim 1. The needle assembly includes a needle cannula having a proximal end and a distal end with a puncture tip, and a hub member having a proximal end and a distal end, supporting the proximal end of the needle cannula. The needle assembly further includes a shield member axially movable with respect to the hub member and the needle cannula, between a non-shielding position in which the puncture tip of the needle cannula is exposed from the shield member, and a shielding position in which the shield member covers the puncture tip of the needle cannula. The needle assembly also includes a pair of extendable members interconnecting the hub member and the shield member. The extendable members include structure which is capable of spreading to form a pair of wings extending from opposing sides of the needle assembly, and extending to cause the shield member to move from the retracted position to the extended position. Desirably, the extendable members are also capable of joining to form a unitary structure, such as for insertion of the needle device. In particular, the pair of extendable members are moveable between a mating position in which the form a unitary structure such as a dorsal fin, a lateral position in which they are spread apart to form a pair of wings extending from opposing sides of the needle assembly, and an extended position in which they are extended to cause the shield member to move from the non-shielding position to the shielding position. Desirably, the pair of extendable members are formed of a resilient flexible material.

Each of the pair of extendable members desirably includes a proximal extension and a distal extension, with each of the proximal extensions being connected to opposing lateral sides of the hub member and each of the distal extensions being connected to opposing lateral sides of the shield member. Such connections desirably act as living hinges. Moreover, each pair of extendable members desirably includes an extendable bridge having structure capable of extending to move the shield member from the non-shielding position to the shielding position. The structure of each of the extendable bridges may further include a partial cut-away portion forming a living hinge, which provides for extension of the bridge and movement of the shield member.

In particularly desirable embodiments, the needle assembly includes means for preventing the shield member from moving in an axial direction between the shielding position and the non-shielding position, by locking the pair of extendable members in the extended position. For example, the extendable bridges may include a locking mechanism, and desirably include first and second locking mechanisms, with the first locking mechanisms engagable with each other and the second locking mechanisms engagable with each other. Alternatively, or in addition thereto, the shield member may include a tip guard assembly for locking the shield member in the shielding position. Such a tip guard assembly may include a tip guard housing formed from a plastic material, and a metallic spring clip being mounted to the housing which is biased against the needle cannula when the shield member is in the non-shielding position and which is resiliently moved over the distal end of the needle cannula when the shield member is in the shielding position.

In a further embodiment, the present invention is directed to a shieldable blood collection set which includes a needle assembly as described above, a flexible tube connected to a proximal end of a hub member of the needle assembly, and a fixture connected to the opposing end of the flexible tube for connecting the blood collection set to a receptacle. In particular, the needle assembly includes a needle cannula, a hub supporting the needle cannula, a shield in axial alignment with the hub and movable between a non-shielding position and a shielding position, and a pair of extendable members movable between a mating position forming a unitary structure, a lateral position forming a pair of wings, and a extended position for causing the shield to move from the non-shielding position to the shielding position.

Desirably, the shield is in the non-shielding position and is adjacent the hub when the pair of extendable members are in the mating position and/or the lateral position, and the shield is in the shielding position when the pair of extendable members are extended to the extended position.

### DESCRIPTION OF THE DRAWINGS

FIG. 1 is a perspective view of a needle assembly in accordance with the present invention, shown in a non-shielding position;

FIG. 2 is a perspective view of the needle assembly of FIG. 1, shown in a sampling position;

FIG. 3 is a perspective view of the needle of FIG. 1, shown in an shielding position;

FIG. 4 is a cross-sectional view taken along line IV-IV of FIG. 3;

FIG. 5 is a cross-sectional view taken along line V-V of FIG. 3;

FIG. 6 is a perspective view of a blood collection set in accordance with the present invention;

FIG. 7 is a perspective view of an alternate needle assembly shown in an shielding position including a tip guard;

FIG. 8 is a cross-sectional view of the needle assembly of FIG. 7;

FIG. 9 is a perspective view of a tip guard assembly for use in an alternate embodiment of the present invention;

FIG. 10 is a cross-sectional view of the tip guard assembly of FIG. 9, shown in a non-shielding position; and

FIG. 11 is a cross-sectional view of the tip guard assembly of FIG. 9, shown in a shielding position.

### DETAILED DESCRIPTION

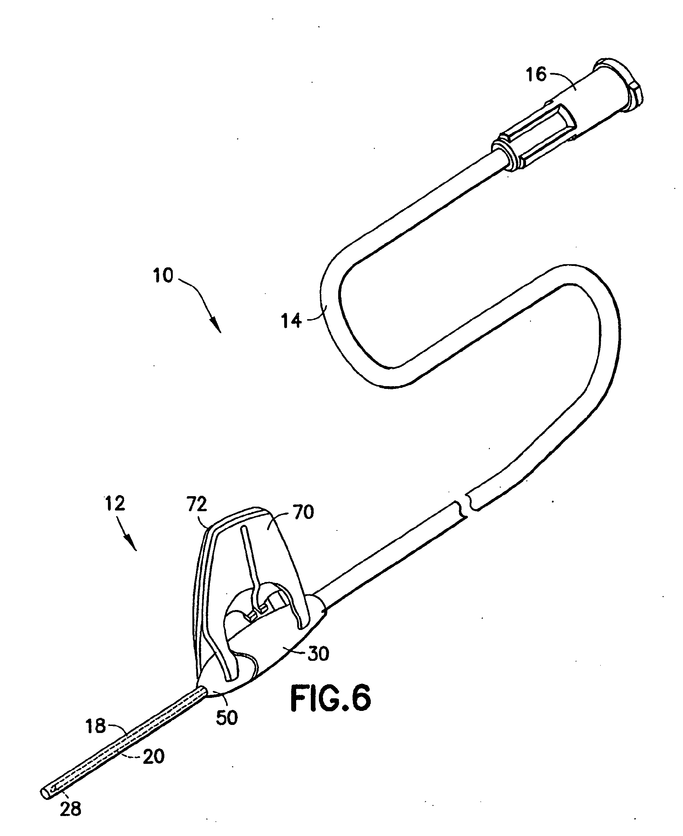

Referring to the drawings in which like reference characters refer to like parts throughout the several views thereof, FIG. 6 illustrates a blood collection set **10** in accordance with the present invention and the related features. The present invention is generally described in terms of a blood collection set, and encompasses such a blood collection set as well as a shieldable needle assembly for use in such a blood collection set.

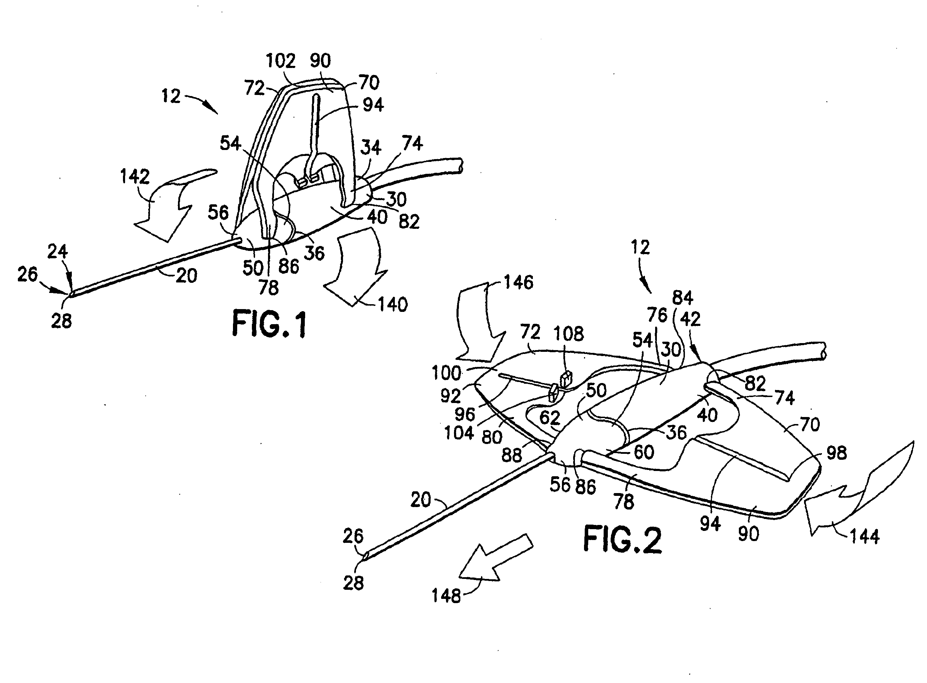

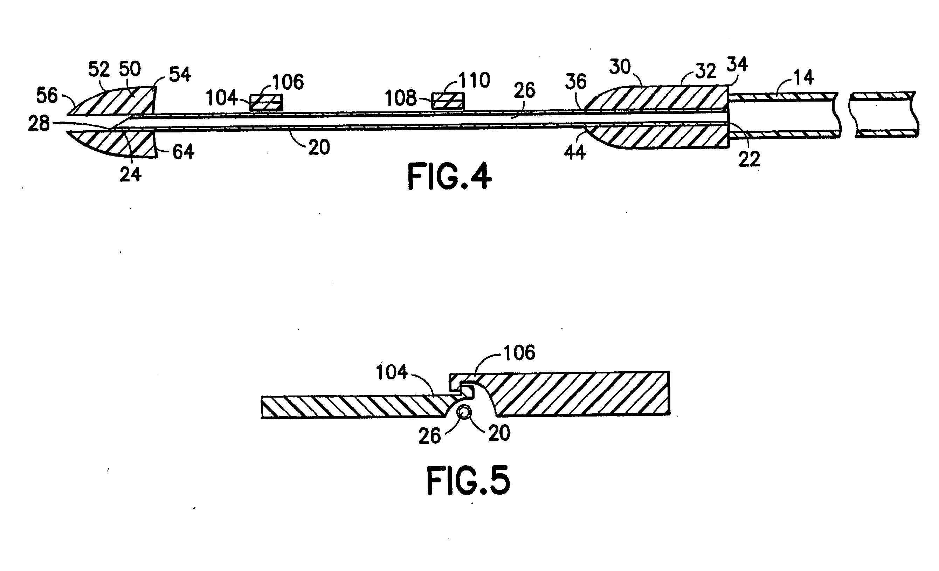

As shown in FIG. 6, blood collection set **10** includes a shieldable needle device **12**, a flexible tube **14** extending from needle device **12**, and a fixture **16** mounted to flexible tube **14**. Shieldable needle device **12** of blood collection set **10** is shown in detail in FIGS. 1-5, and includes a needle cannula **20**, a hub **30**, and a shield **50**. Fixture **16** is connectable to a receptacle (not shown) for use in blood collection procedures, and may therefore include appropriate structure such as a Luer type mating surface, as is known in the art.

Needle cannula **20** includes a proximal end **22** and an opposing distal end **24**, with lumen **26** extending through needle cannula **20** from proximal end **22** to distal end **24**. Distal end **24** of needle cannula **20** is beveled to define a sharp puncture tip **28**, such as an intravenous puncture tip. Puncture tip **28** is provided for insertion into a patient's blood vessel, such as a vein, and is therefore designed to provide ease of insertion and minimal discomfort during venipuncture. A removable protective packaging cover **18** may be positioned over distal end **24** of needle cannula **20** for protection from puncture tip **28** prior to use of blood collection set **10**.

Needle device **12** further includes hub **30.** Hub **30** is a unitary structure, desirably molded from a thermoplastic material. Hub **30** is generally defined by an elongate tubular body in the form of housing **32** having a proximal end **34**, a distal end **36**, and an internal passageway **38** extending therethrough from proximal end **34** to distal end **36**. Needle cannula **20** is positioned within and is supported by internal passageway **38** of hub **30**, with distal end **24** of needle cannula **20** extending from distal end **36** of hub **30**. Desirably, needle cannula **20** and hub **30** are separate parts which are fixedly attached and secured through an appropriate medical grade adhesive or the like.

Proximal end **34** of hub **30** is adapted for connection with a flexible tube **14** of blood collection set **10**. As such, hub **30** may include any appropriate means for coupling with flexible tube **14**, as is known in the art.

Needle device **12** further includes shield **50,** which is defined by a housing **52**. Housing **52** is a unitary structure, desirably molded from a thermoplastic material, including a proximal end **54**, a distal end **56** and an internal passageway **58** extending between proximal end **54** and distal end **56**.

Shield **50** co-axially surrounds a portion of needle cannula **20,** and is positioned axially with respect to hub **30**. In particular, shield **50** is positioned distally of hub **30**, that is, beyond distal end **36** of hub **30** toward distal end **24** of needle cannula **20**. Shield **50** is movable between a non-shielding or retracted position in which puncture tip **28** is exposed from distal end **56** of shield **50**, and a shielding or extended position in which puncture tip **28** of needle cannula **20** is covered by shield **50**, as will be discussed in more detail herein.

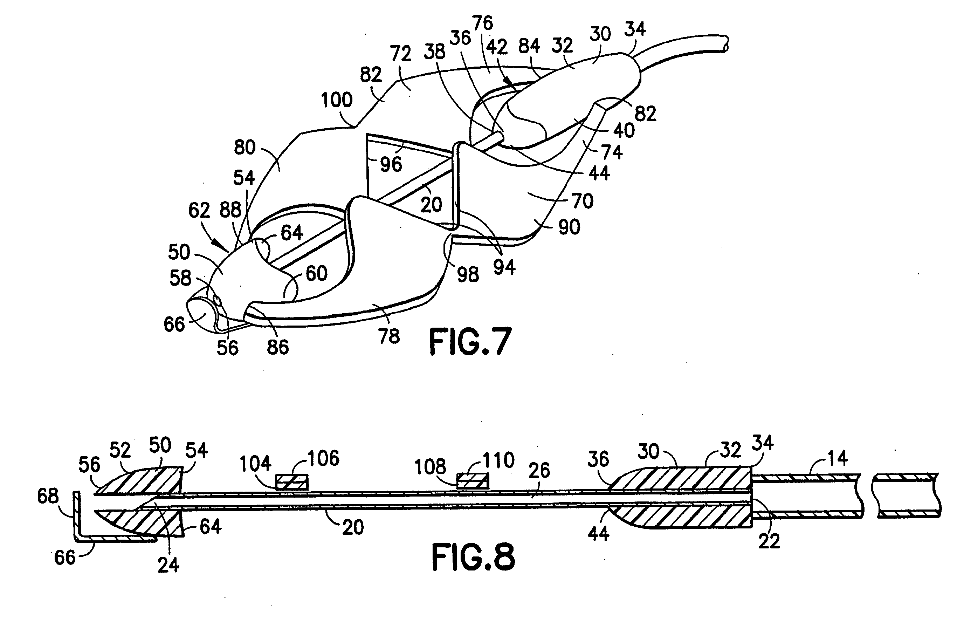

Optionally, as shown in FIGS. 7-8, a needle tip guard **66** is attached to housing **52** of shield **50** toward distal end **56** thereof. Tip guard **66** is provided to automatically cover the opening to internal passageway **58** at distal end **56** of shield **50** when shield **50** is moved to the fully extended position, covering puncture tip **28** of needle cannula **20**. Tip guard **66** may be a curved leaf spring of metal or the like having an axially extending spring leg which curves generally at a right-angle to form locking plate **68**. Tip guard **66** may be attached to housing **52** of shield **50** through any known means, such as through an adhesive, or through the shape and design of the tip guard, such as by a collar which extends around or into housing **52** of shield **50**.

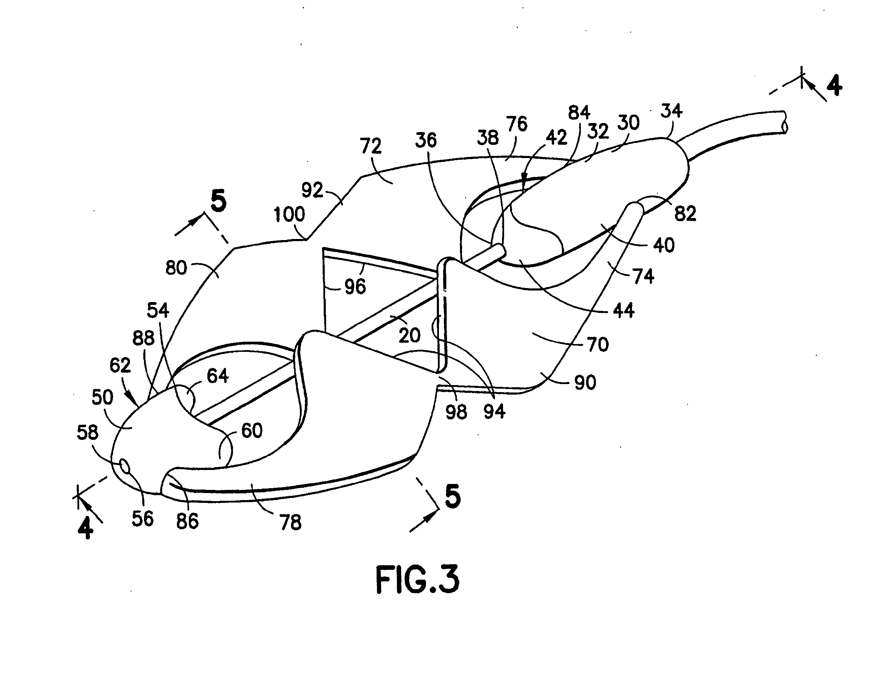

Needle device **12** also includes a pair of extendable members **70** and **72** interconnecting hub **30** and shield **50**. Each of extendable members **70** and **72** includes shape and structure capable of forming at least two distinct positions. More particularly, as shown in FIG. 2, extendable members **70** and **72** are provide in a laterally spaced position forming a pair of wings extending laterally at opposing sides of needle device **12**. Extendable members **70** and **72** are capable of being extended from this laterally spaced position toward the central axis of needle device **12** and toward each other to cause shield **50** to move from the non-shielding position to the shielding position, as shown in FIG. 3.

In one particular embodiment, each of extendable members **70** and **72** includes shape and structure capable of forming three distinct positions. More particularly, in a first mating position, extendable members **70** and **72** can be joined together to form a unitary structure, as shown in FIG. 1. In a second lateral position, extendable members **70** and **72** are spread apart to form a pair of wings extending laterally at opposing sides of needle device **12**, as shown in FIG. 2. In a third extended position, extendable members **70** and **72** are extended to cause shield **50** to move from the non-shielding position to the shielding position, as shown in FIG. 3.

As noted, the shape and structure of extendable members **70** and **72** account for movement between these distinct positions. In particular, extendable members **70** and **72** include proximal extensions **74** and **76**, respectively, and distal extensions **78** and **80**, respectively. Proximal extensions **74** and **76** are separately attached to hub **30** at opposing lateral sides **40** and **42** of hub **30**, respectively. Such attachment of proximal extensions **74** and **76** to hub **30** forms proximal living hinges **82** and **84**, respectively, at the point of attachment of proximal extensions **74** and **76** to hub **30**. In a similar manner, distal extensions **78** and **80** are separately attached to shield **50** at opposing lateral sides **60** and **62** of shield **50**, respectively. Such attachment between distal extensions **78** and **80** to shield **50** forms distal living hinges **86** and **88**, respectively, at the point of attachment of distal extensions **78** and **80** to shield **50**.

Extendable members **70** and **72** further include extendable bridge portions **90** and **92** extending between proximal extensions **74** and **76** and distal extensions **78** and **80**, respectively. Extendable bridges **90** and **92** provide the main body for the interconnection between hub **30** and shield **50**, with proximal extensions **74** and **76** and distal extensions **78** and **80** providing the point of attachment to hub **30** and shield **50**, respectively, for such interconnection. Each of extendable bridges **90** and **92** includes a partial cut-away portion **94** and **96** respectively. These partial cut-away portions **94** and **96** are in the form of a partial slice through the structure of each of extendable bridges **90** and **92**. The partial slices through the structure thereof do not extend entirely therealong, but end at an edge portion, thereby forming two separate hinges in the form of bridge hinges **98** and **100**, respectively.

As noted, such structure permits each of extendable members **70** and **72** to move between at least two, and desirably three distinct positions. In a first mating position shown in FIG. 1, extendable members **70** and **72** are joined together at a top portion of needle device **12** to form a unitary structure, such as a dorsal fin. This is easily accomplished through a bending of proximal extensions **74** and **76** at proximal living hinges **82** and **84**, and distal extensions **78** and **80** at distal living hinges **86** and **88**. This unitary structure provides needle device **12** with a low profile for packaging, thus reducing packaging material and expense. Also, this dorsal fin-shaped unitary structure acts as an insertion guide mechanism during venipuncture and positioning of needle device **12** within a patient, as will be described in further detail with respect to use of the needle device **12**. Extendable members **70** and **72** are capable of being releasably maintained in this mating position. This may be accomplished, for example, by providing a latch mechanism at juncture **102** between extendable members **70** and **72**. Desirably, extendable members **70** and **72** are releasably maintained in this first position by providing a releasable adhesive therebetween at juncture **102**. Such a releasable adhesive can function to maintain extendable members **70** and **72** joined together in the first mating position, with extendable members **70** and **72** capable of being pried apart to release the adhesive connection therebetween.

Each of extendable members **70** and **72** can be moved in opposing circumferential directions about needle device **12** in a direction of arrows **140** and **142**, respectively, thereby causing extendable members to be moved to a lateral position as depicted in FIG. 2. Such movement is accomplished through the hinged attachment of extendable members **70** and **72** to opposing lateral sides **40** and **42** of hub **30** and opposing lateral sides **60** and **62** of shield **50** at proximal living hinges **82** and **84** and distal living hinges **86** and **88**. When in this lateral position, extendable members **70** and **72** act as a pair of stabilizers in the form of wings extending laterally from needle device **12** at opposing sides thereof, providing sampling or blood collection set **10** as a butterfly-type wing assembly. When extendable members **70** and **72** are in this lateral position forming a pair of wings, they are particularly adapted to lie flat against the surface of a patient's skin during the sampling or blood collection procedure, and may be taped to the patient's skin to maintain blood collection set **10** in place.

Alternatively, needle device **12** may be packaged in the position as shown in FIG. 2, with extendable members **70** and **72** laterally spread apart. In this case, extendable members **70** and **72** may be moved together to form a unitary structure as shown in FIG. 1 to assist in needle insertion, and then spread apart to the lateral position of FIG. 2 as described above.

As depicted in FIGS. 1 and 2, shield **50** is desirably located adjacent hub **30** when extendable members **70** and **72** are in the mated position and the lateral position. As such, distal face **44** of hub **30** directly contacts proximal face **64** of shield **50**, thereby forming an axially aligned unitary structure.

Each of extendable members **70** and **72** can further be moved by applying an inward force in a direction of arrows **144** and **146**, thereby causing extendable members **70** and **72** to be extended to an extended position, as shown in FIG. 3. Such extendable movement is accomplished through the cut-away portions **94** and **96** creating bridge hinges **98** and **100**, respectively. By applying force in the direction of arrows **144** and **146**, cut-away portions **94** and **96** are caused to be spread apart, with extendable bridges **90** and **92** extending along bridge hinges **98** and **100**. Since each of extendable members **70** and **72** are connected to hub **30** and shield **50** through proximal living hinges **82** and **84** at proximal extensions **74** and **76** and through distal living hinges **86** and **88** at distal extensions **78** and **80**, such extension along bridge hinges **98** and **100** causes shield **50** to be moved in a direction of arrow **148** along needle cannula **20**, with hub **30** and shield **50** being moved in opposing axial directions. Thus, shield **50** is caused to be moved from the retracted or non-shielding position in which puncture tip **28** of needle cannula **20** is exposed, to the extended or shielding position, in which puncture tip **28** of needle cannula **20** is covered by shield **50**. Moreover, the shape and structure of extendable members **70** and **72** prevents shield **50** from moving entirely beyond puncture tip **28** such that shield **50** is removed from needle cannula **20**, thereby re-exposing puncture tip **28**.

Extendable bridges **90** and **92** further include locking mechanisms for locking the pair of extendable bridges **90** and **92** in place in the extended position. For example, as shown in FIGS. 3-5, first locking mechanisms **104** and **106** may be provided at opposing portions of extendable bridges **90** and **92**, respectively, and second locking mechanisms **108** and **110** may be provided at opposing portions of extendable bridges **90** and **92**, respectively. Moreover, first locking mechanisms **104** and **106**, as well as second locking mechanisms **108** and **110**, include corresponding engaging structures so as to engage each other and lock in place, thereby preventing movement in an opposing direction. During extension of extendable bridges **90** and **92**, first locking mechanisms **104** and **106** move toward each other and engage each other, while second locking mechanisms **108** and **110** also move toward each other and engage each other at a separate location. As such, extendable bridges **90** and **92** engage each other at two separate locking positions, permitting extendable members **70** and **72** to lock at two distinct places, providing improved lock strength for preventing shield **50** from being moved from the non-shielding position to the shielding position.

As shown in FIGS. 3-5, first locking mechanisms **104** and **106** and second locking mechanisms **108** and **110** may engage each other, respectively, at a position above needle cannula **20**. Alternatively, extendable members **70** and **72** may be designed to include locking mechanisms which engage at any position, such as below needle cannula **20**, or straddling needle cannula **20** about opposing sides thereof. In a further embodiment, it is contemplated that locking mechanisms may be provided on one or both of extendable members **70** and **72** for engagement directly with needle cannula **20**.

Needle device **12** of blood collection set **10** may be constructed as a unitary member which is integrally formed including hub **30**, shield **50**, and extendable members **70** and **72** extending therebetween, with appropriate distinction between each of the elements as discussed above and shown in FIGS. 1-3. Alternatively, hub **30**, shield **50**, and extendable members **70** and **72** may be separately formed, and joined together through an appropriate adhesive. Desirably, each element is constructed of a resilient material, with extendable members **70** and **72** being constructed of a resiliently flexible material, thus providing appropriate flexibility for hinged movement of proximal living hinges **82** and **84**, distal living hinges **86** and **88**, and bridge hinges **98** and **100**. Desirably, the components are molded of a thermoplastic polymeric material.

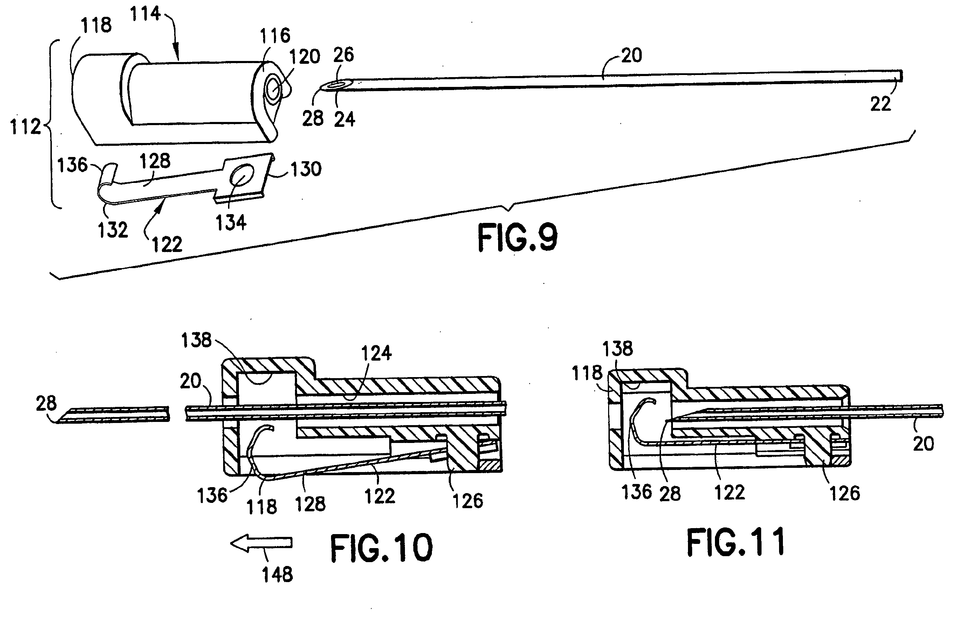

As noted above, shield **50** may optionally include tip guard **66,** for covering puncture tip **28** of needle cannula **20** when shield **50** is moved to the fully extended position, as shown in FIGS. 7 and 8. In a further embodiment of the present invention depicted in FIGS. 9-11, shield **50** may be provided in the form of tip guard assembly **112**, or may be provided as shield **50** including tip guard assembly **112** as a portion thereof, for engagement with puncture tip **28** when shield **50** is moved to the extended position. Tip guard assembly **112** includes a housing **114** and a protective clip **122**. Housing **114** is a unitary structure, desirably molded from a thermoplastic material, including a proximal end **116,** a distal end **118,** and an internal passage **120** extending between the ends. Portions of internal passage **120** adjacent distal end **118** define an enlarged clip receptacle **124**, as shown in FIG. 8. A clip mounting post **126** extends downwardly from housing **114** at a location near proximal end **116** of housing **114**.

Protective clip **122** is unitarily stamped and formed from a resiliently deflectable metallic material. Protective clip **122** includes a planar spring leg **128** with a proximal end **130** and an opposed distal end **132**. A mounting aperture **134** extends through spring leg **128** at a location near proximal end **130**. Mounting aperture **134** has a diameter approximately equal to or slightly less than the diameter of mounting post **126** of housing **114.** As such, mounting post **126** can be forced through mounting aperture **134** when the axis of mounting post **126** and the axis of mounting aperture **134** are substantially collinear. A lock out leg **136** extends angularly from distal end **132** of spring leg **128**. Lock out leg **136** is bent back toward proximal end **130** of clip **126**. The bends in lock out leg **136** enable secure protective engagement with puncture tip **28** of needle cannula **20** and further enables smooth axial sliding movement of tip guard assembly **112** along needle cannula **20**.

Blood collection set **10** can be packaged substantially in the condition shown in FIG. 6. The shape and configuration of needle device **12** provides a low profile for packaging of blood collection set **10**.

In use, blood collection set **10** is provided with needle device **12** assembled as described and including flexible tube **14** extending from needle device **12** and connected to fixture **16**. After removing blood collection set **10** from its package, it can be assembled with other appropriate medical equipment for use. For example, an appropriate receptacle, such as a non-patient needle assembly and a needle holder, may be connected to blood collection set **10** through fixture **16**, thereby providing fluid communication with lumen **26** through needle cannula **20**.

To prepare for use of blood collection set **10**, the user grasps blood collection set **10** at needle device **12** and removes the protective packaging cover **18** to expose puncture tip **28** of needle cannula **20**. The medical practitioner can then urge puncture tip **28** at distal end **24** of needle cannula **20** into a targeted blood vessel of a patient. During such positioning, extendable members **70** and **72** of needle device **12** may be in the mating position forming a unitary structure as shown in FIG. 1 and described above. As such, the user can grasp needle device **12** at opposing sides of extendable members **70** and **72** between the user's thumb and forefinger, to facilitate positioning and placing of needle device **12** with one hand.

After venipuncture is accomplished, the user can then release the connection at juncture **102**, such as by prying apart extendable members **70** and **72**, if they are adhesively joined or locked together. Extendable members **70** and **72** can then be moved in opposing circumferential directions about needle device **12** in a direction of arrows **140** and **142**, through the action of proximal living hinges **82** and **84** at opposing lateral sides **40** and **42** of hub **30** and distal living hinges **86** and **88** at opposing lateral sides **60** and **62** of shield **50**. Such spreading apart causes extendable members **70** and **72** to form a pair of wings extending from opposing lateral sides of needle device **12**. These wings formed by extendable members **70** and **72** can then be taped to the patient's skin to maintain blood collection set **10** in place during the procedure. Alternatively, extendable members **70** and **72** may automatically flex to the lateral position shown in FIG. 2 based on the resilient nature of proximal living hinges **82** and **84** at opposing lateral sides **40** and **42** of hub **30** and distal living hinges **86** and **88** at opposing lateral sides **60** and **62** of shield **50**.

Upon completion of the desired procedure, such as when all desired samples have been drawn or after infusion has occurred, needle cannula **20** is withdrawn from the patient. Since extendable members **70** and **72** are connected to hub **30** and shield **50** through proximal living hinges **82** and **84** and distal living hinges **86** and **88**, extendable members **70** and **72** can be brought together by moving them about proximal living hinges **82** and **84** and distal living hinges **86** and **88** in a direction opposite arrows **140** and **142** between the user's thumb and forefinger, to again form a unitary dorsal fin structure, as in FIG. 1. Needle cannula **20** can then be withdrawn from the patient.

After removal of needle cannula **20** from the patient, activation of the safety feature of needle device **12** is accomplished. In particular, the user releases the needle device **12** from the user's thumb and forefinger. The resilient nature of the structure of needle device **12** and extendable members **70** and **72** cause them to move in the direction of arrows **140** and **142**, and return to the lateral position forming a pair of wings extending from opposing sides of needle assembly **12**.

At this point, the user can grasp extendable members **70** and **72** at the opposing edges adjacent bridge hinges **98** and **100**, between the user's thumb and forefinger. By exerting a force in the direction of arrows **144** and **146** against bridge hinges **98** and **100,** i.e., by movement of bridge hinges **98** and **100** toward each other toward the central axis of needle device **10**, cut-away portions **94** and **96** are expanded across bridge hinges **98** and **100**, thereby causing extendable bridges **90** and **92** to extend. With the interconnection between hub **30** and shield **50** established through extendable members **70** and **72**, such extension along bridge hinges **98** and **100** causes shield **50** to be moved in a direction of arrow **148** along needle cannula **20**, with hub **30** and shield **50** being moved in opposing axial directions. Hence, puncture tip **28** of needle cannula **20** is safely shielded by shield **50** in the shielding position as shown in FIG. **3**.

Extension of extendable bridges **90** and **92** further causes first locking mechanisms **104** and **106** and second locking mechanisms **108** and **110** to move toward each other and engage each other at two separate locking positions, thereby locking extendable members **70** and **72** in place to prevent movement in an opposing direction. Furthermore, in embodiments in which shield **50** incorporates a tip guard **66**, as shown in FIGS. 7-8 described above, locking plate **68** of tip guard **66** is biased against needle cannula **20** when shield **50** is in the non-shielding position. As shield **50** is extended toward distal end **24** of needle cannula **20**, tip guard **66** slides along a bottom surface of needle cannula **20** until shield **50** passes puncture tip **28**. At this point, locking plate **68** is no longer in engagement with needle cannula **20**, and is caused to automatically extend over the distal end **56** of shield **50**, thereby preventing any re-emergence of puncture tip **28**, should the locking mechanisms of extendable members **70** and **72** become disengaged. In this manner, tip guard **66** acts as a further lock for preventing exposure of puncture tip **28**. Tip guard assembly **112**, shown in FIGS. 9-11 as discussed above, operates in a similar manner, with movement of shield **50** to the shielding position causing protective clip **122** to extend beyond puncture tip **28** of needle cannula **20**, thereby further preventing re-exposure of puncture tip **28**. Blood collection set **10** may then be appropriately discarded.

While the needle assembly of the present invention has been described in terms of one embodiment for use in connection with a blood collection system, it is further contemplated that the needle assembly could be used with other medical procedures, such as in conjunction with a conventional intravenous infusion set, which are well known in the art for use with needle assemblies.

While the present invention is satisfied by embodiments in many different forms, there is shown in the drawings and described herein in detail, the preferred embodiments of the invention, with the understanding that the present disclosure is to be considered as exemplary of the principles of the invention and is not intended to limit the invention to the embodiments illustrated. Various other embodiments will be apparent to and readily made by those skilled in the art without departing from the scope of the invention. The scope of the invention will be measured by the appended claims and their equivalents.

## Claims

1. Needle assembly, comprising:
a needle cannula (20) having a proximal end (22) and a distal end (24) with a puncture tip (28);
a hub member (30) having a proximal end (34) and a distal end (36), said hub member (30) supporting the proximal end (22) of the needle cannula (20);
a shield member (50) axially movable with respect to said hub member (30) and said needle cannula (20) between a non-shielding position in which said puncture tip (28) of said needle cannula (20) is exposed from said shield member (50), and a shielding position in which said shield member (50) covers said puncture tip (28) of said needle cannula (20);
a pair of extendable members (70, 72) interconnecting the hub member (30) and the shield member (50), said pair of extendable members (70, 72) movable between a lateral position in which said pair of extendable members (70, 72) extend from opposing sides of said needle assembly, and an extended position in which said pair of extendable members (70, 72) are extended to cause said shield member (50) to move from said non-shielding position to said shielding position,
**characterized in that**
the pair of extendable members (70,72) form a pair of wings extending from opposing sides of said needle assembly when in said lateral position and the pair of extendable members (70, 72) are movable between a mating position in which said pair of extendable members (70, 72) form a unitary structure, said lateral position and said extended position.

2. A needle assembly as in claim 1, wherein said pair of extendable members (70, 72) are formed of a resilient flexible material.

3. A needle assembly as in claim 1, wherein each of said pair of extendable members (70, 72) includes a proximal extension (74, 76) and a distal extension (78, 80), each of said proximal extensions (74, 76) of each of said pair of extendable members (70, 72) connected to opposing lateral sides (40, 42) of said hub member (30) and each of said distal extensions (78, 80) of each of said pair of extendable members (70, 72) connected to opposing lateral sides (60, 62) of said shield member (50).

4. A needle assembly as in claim 3, wherein each of said proximal extensions (74, 76) of each of said pair of extendable members (70, 72) are connected to opposing lateral sides (40,42) of said hub member (30) and each of said distal extensions (78, 80) of each of said pair of extendable members (70, 72) are connected to opposing lateral sides (60, 62) of said shield member (50) through living hinges (86, 88).

5. A needle assembly as in claim 3, wherein each of said pair of extendable members (70, 72) includes an extendable bridge (90, 92) having structure capable of extending to move said shield member (50) from said non-shielding position to said shielding position.

6. A needle assembly as in claim 5, wherein said structure of each of said extendable bridges (90, 92) includes a partial cut-away portion (94, 96) forming a living hinge (98, 100).

7. A needle assembly as in claim 6, wherein each of said extendable bridges (90, 92) includes first and second locking mechanisms( 104, 106, 108, 110), said first locking mechanisms (104, 106) engagable with each other and said second locking mechanisms (108, 110) engagable with each other for locking said pair of extendable members (70, 72) in said extended position in which said pair of extendable members (70, 72) are extended.

8. A needle assembly as in claim 1, wherein in the lateral position the pair of extendable members (70, 72) are spread apart to form a pair of wings.

9. A needle assembly as in claim 8, wherein said pair of extendable members (70, 72) form a dorsal fin when said pair of extendable members (70, 72) are in said position forming a unitary structure.

10. A needle assembly as in claim 8, wherein said pair of extendable members (70, 72) includes means for releasably maintaining said pair of extendable members (70, 72) in said position in which said pair of extendable members (70, 72) form a unitary structure.

11. A needle assembly as in any of the preceeding claims, further comprising means for preventing said shield member (50) from moving in an axial direction between said shielding position and said non-shielding position.

12. A needle assembly as in any of the preceeding claims, wherein said shield member (50) further comprises a tip guard (112) including a tip guard housing (114) formed from a plastic material, a metallic spring clip (122) being mounted to said housing (114), said spring clip (122) being biased against said needle cannula (20) when shield member (50) is in said non-shielding position and being resiliently moved over said distal end of said needle cannula (20) when said shield member (50) is in said shielding position.

13. A needle assembly as in any of the preceeding claims, wherein said hub member (30) is adapted for connection to a flexible tube (14) of a blood collection set.

14. A needle assembly as in claim 8, wherein said shield member (50) is in said non-shielding position and is adjacent said hub member (30) when said pair of extendable members (70, 72) are in said mating or joined position and said lateral or spread position, and said shield member (50) is in said shielding position when said pair of extendable members (70, 72) are extended to said extended position.

## Patentansprüche

1. Nadelanordnung, umfassend:
- eine Nadelkanüle (20), die ein proximales Ende (22) und ein distales Ende (24) mit einer Punktionsspitze (28) hat;
- ein Nabenelement (30), das ein proximales Ende (34) und ein distales Ende (36) hat, wobei das besagte Nabenelement (30) das proximale Ende (22) der Nadelkanüle (20) stützt;
- ein Schirmelement (50), das mit Bezug zu dem besagten Nabenelement (30) und zu der besagten Nadelkanüle (20) axial zwischen einer nicht-schirmenden Position, in der die besagte Punktionsspitze (28) der besagten Nadelkanüle (20) von dem besagten Schirmelement (50) frei gelegt ist, und einer schirmenden Position bewegt werden kann, in der das besagte Schirmelement (50) die besagte Punktionsspitze (28) der besagten Nadelkanüle (20) bedeckt;
- einem Paar von erweiterbaren Elementen (70, 72), die das Nabenelement (30) und das Schirmelement (50) miteinander verbinden, wobei das besagte Paar von erweiterbaren Elementen (70, 72) zwischen einer lateralen Position, in der sich das besagte Paar von erweiterbaren Elementen (70, 72) von den gegenüberliegenden Seiten der besagten Nadelanordnung ausdehnt, und einer erweiterten Position bewegt werden kann, in der das besagte Paar von erweiterbaren Elementen (70, 72) erweitert ist, so dass verursacht wird, dass sich das besagte Schirmelement (50) aus der besagten nicht-schirmenden Position in die besagte schirmende Position bewegt;
**dadurch gekennzeichnet, dass:**
- das Paar von erweiterbaren Elementen (70, 72) ein Paar von Flügeln bildet, das sich von den gegenüberliegenden Seiten der besagten Nadelanordnung ausdehnt, wenn es sich in der besagten lateralen Position befindet, und das Paar von erweiterbaren Elementen (70, 72) zwischen einer ineinander greifenden Position, in der das besagte Paar von erweiterbaren Elementen (70, 72) eine einheitliche Struktur bildet, der besagten lateralen Position und der besagten erweiterten Position bewegt werden kann.

2. Eine Nadelanordnung wie in dem Anspruch 1, wobei das besagte Paar von erweiterbaren Elementen (70, 72) aus einem belastbaren flexiblen Material gebildet ist.

3. Eine Nadelanordnung wie in dem Anspruch 1, wobei jedes erweiterbare Element aus dem besagten Paar von erweiterbaren Elementen (70, 72) einen proximalen Fortsatz (74, 76) und einen distalen Fortsatz (78, 80) aufweist, wobei jeder der proximalen Fortsätze (74, 76) von jedem erweiterbaren Element aus dem besagten Paar von erweiterbaren Elementen (70, 72) mit den gegenüberliegenden lateralen Seiten (40, 42) des besagten Nabenelements (30) verbunden ist, und wobei jeder der distalen Fortsätze (78, 80) von jedem erweiterbaren Element aus dem besagten Paar von erweiterbaren Elementen (70, 72) mit den gegenüberliegenden lateralen Seiten (60, 62) des besagten Schirmelements (50) verbunden ist.

4. Eine Nadelanordnung wie in dem Anspruch 3, wobei jeder der proximalen Fortsätze (74, 76) von jedem erweiterbaren Element aus dem besagten Paar von erweiterbaren Elementen (70, 72) mit den gegenüberliegenden lateralen Seiten (40, 42) des besagten Nabenelements (30) verbunden ist, und wobei jeder der distalen Fortsätze (78, 80) von jedem erweiterbaren Element aus dem besagten Paar von erweiterbaren Elementen (70, 72) mit den gegenüberliegenden lateralen Seiten (60, 62) des besagten Schirmelements (50) über ein Filmscharnier (86, 88) verbunden ist.

5. Eine Nadelanordnung wie in dem Anspruch 3, wobei jedes erweiterbare Element aus dem besagten Paar von erweiterbaren Elementen (70, 72) eine erweiterbare Brücke (90, 92) aufweist, die eine Struktur hat, die in der Lage ist, sich auszudehnen, um das besagte Schirmelement (50) aus der besagten nicht-schirmenden Position in die besagte schirmende Position zu bewegen.

6. Eine Nadelanordnung wie in dem Anspruch 5, wobei die besagte Struktur von jeder der erweiterbaren Brücken (90, 92) einen zu einem Teil weg geschnittenen Anteil (94, 96) aufweist, der ein Filmscharnier (98, 100) bildet.

7. Eine Nadelanordnung wie in dem Anspruch 6, wobei jede der erweiterbaren Brücken (90, 92) erste und zweite Arretierungsmechanismen (104, 106, 108, 110) aufweist, wobei die besagten ersten Arretierungsmechanismen (104, 106) miteinander in Eingriff gebracht werden können und wobei die besagten zweiten Arretierungsmechanismen (108, 110) miteinander in Eingriff gebracht werden können, um das besagte Paar von erweiterbaren Elementen (70, 72) in der besagten erweiterten Position zu arretieren, in der das besagte Paar von erweiterbaren Elementen (70, 72) erweitert ist.

8. Eine Nadelanordnung wie in dem Anspruch 1, wobei in der lateralen Position das Paar von erweiterbaren Elementen (70, 72) auseinander gespreizt sind, um ein Paar von Flügeln zu bilden.

9. Eine Nadelanordnung wie in dem Anspruch 8, wobei das besagte Paar von erweiterbaren Elementen (70, 72) dann eine Rückenflosse bildet, wenn sich das besagte Paar von erweiterbaren Elementen (70, 72) in der besagten Position befindet, in der es eine einheitliche Struktur bildet.

10. Eine Nadelanordnung wie in dem Anspruch 8, wobei das besagte Paar von erweiterbaren Elementen (70, 72) Mittel aufweist, die dazu dienen, dass das besagte Paar von erweiterbaren Elementen (70, 72) freigebbar in der besagten Position zu halten, in der das besagte Paar von erweiterbaren Elementen (70, 72) eine einheitliche Struktur bildet.

11. Eine Nadelanordnung wie in einem der vorhergehenden Ansprüche, wobei sie des Weiteren Mittel umfasst, die dazu dienen, dass verhindert wird, dass sich das besagte Schirmelement (50) in einer axialen Richtung zwischen der besagten schirmenden Position und der besagten nicht-schirmenden Position bewegt.

12. Eine Nadelanordnung wie in einem der vorhergehenden Ansprüche, wobei das besagte Schirmelement (50) des Weiteren einen Spitzenschutz (112) umfasst, der ein Spitzenschutz-Gehäuse (114), das aus einem Kunststoffmaterial gebildet ist, und einen metallischen Federbügel (122) aufweist, der an dem besagten Gehäuse (114) angebracht ist, wobei der besagte Federbügel (122) dann gegen die besagte Nadelkanüle (20) vorgespannt ist, wenn sich das Schirmelement (50) in der besagten nicht-schirmenden Position befindet, und dann federnd über das besagte distale Ende der besagten Nadelkanüle (20) bewegt wird, wenn sich das Schirmelement (50) in der besagten schirmenden Position befindet.

13. Eine Nadelanordnung wie in einem der vorhergehenden Ansprüche, wobei das besagte Nabenelement (30) für eine Verbindung mit einem flexiblen Schlauch (14) eines Blutspende-Satzes angepasst ist.

14. Eine Nadelanordnung wie in dem Anspruch 8, wobei sich das besagte Schirmelement (50) dann in der besagten nicht-schirmenden Position befindet und mit dem besagten Nabenelement (30) benachbart ist, wenn sich das besagte Paar von erweiterbaren Elementen (70, 72) in der besagten ineinander greifenden oder zusammengefügten Position und in der besagten lateralen oder gespreizten Position befindet, und wobei sich das besagte Schirmelement (50) dann in der besagten schirmenden Position befindet, wenn das besagte Paar von erweiterbaren Elementen (70, 72) in die besagte erweiterte Position erweitert ist.

## Revendications

1. Assemblage d'aiguille, comprenant :
une canule (20) ayant une extrémité proximale (22) et une extrémité distale (24) avec une pointe de ponction (28) ;
un élément de raccord (30) ayant une extrémité proximale (34) et une extrémité distale (36), ledit élément de raccord (30) supportant l'extrémité proximale (22) de la canule (20) de l'aiguille ;
un élément de protection (50) pouvant être déplacé axialement par rapport audit élément de raccord (30) et à ladite canule (20) de l'aiguille entre une position non protégée, dans laquelle ladite pointe de ponction (28) de ladite canule (20) de l'aiguille est exposée par ledit élément de protection (50), et une position protégée, dans laquelle ledit élément de protection (50) recouvre ladite pointe de ponction (28) de ladite canule (20) de l'aiguille ;
une paire d'éléments extensibles (70, 72) raccordant entre eux l'élément de raccord (30) et l'élément de protection (50), ladite paire d'éléments extensibles (70, 72) pouvant se déplacer entre une position latérale, dans laquelle lesdits deux éléments extensibles (70, 72) s'étendent des côtés opposés dudit assemblage d'aiguille, et une position déployée, dans laquelle ladite paire d'éléments extensibles (70, 72) est déployée pour amener ledit élément de protection (50) à se déplacer de ladite position non protégée à ladite position protégée,
**caractérisé en ce que**
la paire d'éléments extensibles (70, 72) forme une paire d'ailes s'étendant des côtés opposés dudit assemblage d'aiguille lorsqu'elle se trouve dans ladite position latérale et la paire d'éléments extensibles (70, 72) peut être déplacée entre une position d'accouplement, dans laquelle elle forme une structure unitaire, ladite position latérale et ladite position déployée.

2. Assemblage d'aiguille selon la revendication 1, dans lequel ladite paire d'éléments extensibles (70, 72) est formée d'un matériau élastique flexible.

3. Assemblage d'aiguille selon la revendication 1, dans lequel chacun des éléments de ladite paire d'éléments extensibles (70, 72) comprend une extension proximale (74, 76) et une extension distale (78, 80), chacune desdites extensions proximales (74, 76) de chacun des éléments de ladite paire d'éléments extensibles (70, 72) étant raccordée aux côtés latéraux opposés (40, 42) dudit élément de raccord (30) et chacune desdites extensions distales (78, 80) de chacun des éléments de ladite paire d'éléments extensibles (70, 72) étant raccordée aux côtés latéraux opposés (60, 62) dudit élément de protection (50).

4. Assemblage d'aiguille selon la revendication 3, dans lequel chacune desdites extensions proximales (74, 76) de chacun des éléments de ladite paire d'éléments extensibles (70, 72) est raccordée aux côtés latéraux opposés (40, 42) dudit élément de raccord (30) et chacune desdites extensions distales (78, 80) de chacun des éléments de ladite paire d'éléments extensibles (70, 72) est raccordée aux côtés latéraux opposés (60, 62) dudit élément de protection (50) par le biais d'articulations vives (86, 88).

5. Assemblage d'aiguille selon la revendication 3, dans lequel chacun des éléments de ladite paire d'éléments extensibles (70, 72) comprend un pont extensible (90, 92) ayant une structure capable de se déployer pour déplacer ledit élément de protection (50) de ladite position non protégée à ladite position protégée.

6. Assemblage d'aiguille selon la revendication 5, dans lequel ladite structure de chacun desdits ponts extensibles (90, 92) comprend une partie partiellement découpée (94, 96) formant une articulation vive (98, 100).

7. Assemblage d'aiguille selon la revendication 6, dans lequel chacun desdits ponts extensibles (90, 92) comprend des premiers et seconds mécanismes de verrouillage (104, 106, 108, 110), lesdits premiers mécanismes de verrouillage (104, 106) pouvant s'engager entre eux et lesdits seconds mécanismes de verrouillage (108, 110) pouvant s'engager entre eux afin de verrouiller ladite paire (70, 72) dans ladite position déployée dans laquelle ladite paire d'éléments extensibles (70, 72) est déployée.

8. Assemblage d'aiguille selon la revendication 1, dans lequel, dans la position latérale, la paire d'éléments extensibles (70, 72) est écartée pour former une paire d'ailes.

9. Assemblage d'aiguille selon la revendication 8, dans lequel ladite paire d'éléments extensibles (70, 72) forme une ailette dorsale lorsqu'elle se trouve dans ladite position formant une structure unitaire.

10. Assemblage d'aiguille selon la revendication 8, dans lequel ladite paire d'éléments extensibles (70, 72) comprend des moyens pour se maintenir de manière libérable dans ladite position dans laquelle ladite paire d'éléments extensibles (70, 72) forme une structure unitaire.

11. Assemblage d'aiguille selon l'une quelconque des revendications précédentes, comprenant en outre des moyens pour empêcher ledit élément de protection (50) de se déplacer en direction axiale entre ladite position protégée et ladite position non protégée.

12. Assemblage d'aiguille selon l'une quelconque des revendications précédentes, dans lequel ledit élément de protection (50) comprend en outre un capot de pointe (112) comprenant un logement de capot de pointe (114) formé d'un matériau plastique, une agrafe élastique métallique (122) étant montée sur ledit logement (114), ladite attache à ressort (122) étant pressée contre ladite canule (20) de l'aiguille lorsque l'élément de protection (50) est dans ladite position non protégée et étant déplacée de manière élastique sur ladite extrémité distale de ladite canule (20) de l'aiguille (20) lorsque ledit élément de protection (50) est dans ladite position protégée.

13. Assemblage d'aiguille selon l'une quelconque des revendications précédentes, dans lequel ledit élément de raccord (30) est adapté pour être raccordé à un tube flexible (14) d'un ensemble de prélèvement de sang.

14. Assemblage d'aiguille selon la revendication 8, dans lequel ledit élément de protection (50) est dans ladite position non protégée et est adjacent audit élément de raccord (30) lorsque ladite paire d'éléments extensibles (70, 72) est dans ladite position d'accouplement ou d'assemblage et dans ladite position latérale ou écartée et ledit élément de protection (50) est dans ladite position protégée lorsque ladite paire d'éléments extensibles (70, 72) est déployée dans ladite position déployée.
